(19) Europäisches Patentamt
European Patent Office
Office européen des brevets

(11) Veröffentlichungsnummer: **0 559 013 B1**

(12) **EUROPÄISCHE PATENTSCHRIFT**

(45) Veröffentlichungstag der Patentschrift: **09.08.95**

(51) Int. Cl.$^6$: **A61K 7/00**, A61K 9/113, B01F 17/00

(21) Anmeldenummer: **93102607.4**

(22) Anmeldetag: **19.02.93**

(54) **Flüssige oder pastöse, lagerstabile, multiple Emulsion des Typs O/W/O.**

(30) Priorität: **04.03.92 DE 4206732**

(43) Veröffentlichungstag der Anmeldung:
**08.09.93 Patentblatt 93/36**

(45) Bekanntmachung des Hinweises auf die Patenterteilung:
**09.08.95 Patentblatt 95/32**

(84) Benannte Vertragsstaaten:
**BE DE ES FR GB IT NL**

(56) Entgegenhaltungen:
**EP-A- 0 176 884**
**EP-A- 0 386 507**
**EP-A- 0 391 124**
**EP-A- 0 459 705**

**DATABASE WPIL Week 8622, Derwent Publications Ltd., London, GB; AN 86-140044**

(73) Patentinhaber: **Th. Goldschmidt AG**
**Goldschmidtstrasse 100**
**D-45127 Essen (DE)**

(72) Erfinder: **Grüning, Burghard, Dr.**
**Waldsaum 11**
**W-4300 Essen 1 (DE)**
Erfinder: **Hameyer, Peter**
**Semperstrasse 5**
**W-4300 Essen 1 (DE)**
Erfinder: **Weitemeyer, Christian, Dr.**
**Sundernholz 67**
**W-4300 Essen 1 (DE)**

EP 0 559 013 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine flüssige oder pastöse, lagerstabile, multiple Emulsion des Typs $O^1/W/O^2$, wobei $O^2$ die äußere Phase der multiplen Emulsion bildet, an der Grenzfläche $O^1/W$ ein hydrophiler Emulgator und an der Grenzfläche $W/O^2$ ein hydrophober Emulgator vorhanden ist.

Multiple Emulsionen des Typs O/W/O haben insbesondere in der Kosmetik, aber auch in manchen technischen Anwendungen an Bedeutung gewonnen. So werden beispielsweise multiple Emulsionen dann angestrebt, wenn zwei oder mehrere öllösliche Substanzen in der Emulsion voneinander getrennt vorliegen sollen, um deren Interaktion miteinander zu vermeiden, oder wenn man in der inneren öligen Phase eine Substanz löst, die beispielsweise vor Luftsauerstoff geschützt sein soll.

Kosmetische Zubereitungen dieses Typs sind in der europäischen Patentanmeldung 0 391 124 beschrieben. Gegenstand der Patentanmeldung ist dabei eine Emulsion, die dadurch erhalten wird, daß man zunächst eine ölige Phase, welche 1 bis 30 Gew.-% (bezogen auf Gesamtzubereitung) eines bei 25 °C festen Fettes und 0,01 bis 5 Gew.-% eines hydrophilen Emulgators enthält, mit Wasser, welches ein wasserlösliches Feuchthaltemittel enthält, in eine O/W-Emulsion überführt und dann diese O/W-Emulsion mit einer zweiten Ölphase, welche die kontinuierliche äußere Phase darstellt, mischt, wobei diese äußere Phase 10 bis 70 Gew.-% beträgt und ein bei 25 °C flüssiges Öl ist und 0,5 bis 10 Gew.-% eines hydrophoben Emulgators enthält. Diese in der europäischen Patentanmeldung 0 391 124 beschriebene multiple Emulsion enthält somit in der inneren Phase eine bei Raumtemperatur feste Ölkomponente. Hierdurch wird eine gewisse Stabilität der Emulsion erreicht und Entmischungen und/oder Wechselwirkungen der Emulgatoren miteinander, welche eine Instabilität der Emulsion verursachen würden, vorgebeugt.

Die Erfindung befaßt sich deshalb insbesondere mit dem Problem, multiple Emulsionen des Typs $O^1/W/O^2$ bereitzustellen, deren innere ölige Phase $O^1$ flüssig ist und wobei die multiplen Emulsionen trotz dieser inneren öligen Phase eine hohe Lagerstabilität aufweisen. Dabei ist der Begriff der Lagerstabilität so zu verstehen, daß die Emulsionen während einer Lagerzeit von drei Monaten bei Raumtemperatur und bei 40 °C keine Veränderungen ihres Verteilungszustandes erkennen lassen.

Überraschenderweise wurde gefunden, daß man flüssige oder pastöse, lagerstabile, multiple Emulsionen des Typs $O^1/W/O^2$, wobei $O^2$ die äußere Phase der multiplen Emulsion bildet, an der Grenzfläche $O^1/W$ ein hydrophiler Emulgator und an der Grenzfläche $W/O^2$ ein hydrophober Emulgator vorhanden ist, dann erhält, wenn erfindungskennzeichnend

(1) der hydrophobe Emulgator einen HLB-Wert $\leqq 8$ aufweist und
  - $a_1$) ein Polyacrylsäureester mit langkettigen Kohlenwasserstoff- und Polyoxyalkylengruppen oder
  - $a_2$) ein Polyoxyalkylenpolysiloxan mit an Si-Atomen gebundenen langkettigen Alkylresten ist, und

(2) der hydrophile Emulgator ein nichtionogener Emulgator mit einem HLB-Wert > 8 ist.

Der Begriff des HLB-Wertes ist gemäß vorliegender Erfindung als experimentell zu bestimmender Vergleichswert anzusehen. Er wird z.B. in dem Aufsatz von W.C. Griffin "Classification of surface-active agents by HLB" in J. Soc. Cosmetic Chemists 1, 311 (1950), näher erläutert.

Der hydrophobe Emulgator:

Dieser ist zunächst durch seinen HLB-Wert gekennzeichnet. Er soll $\leqq 8$, vorzugsweise $\leqq 6$ sein. Dabei wird der hydrophobe Emulgator erfindungsgemäß aus zwei Gruppen von polymeren Verbindungen ausgewählt:

$a_1$) Polyacrylsäureester mit langkettigen Kohlenwasserstoff- und Polyoxyalkylengruppen,
$a_2$) Polyoxyalkylenpolysiloxane mit an Si-Atomen gebundenen langkettigen Alkylresten.

Die hydrophoben Emulgatoren der Gruppe $a_1$) sind in der DE-PS 39 06 702 beschrieben. Sie sind erhältlich durch Umesterung von durch radikalische Polymerisation erhaltenen Polyacrylsäurealkylestern, deren Alkylgruppen 1 bis 4 Kohlenstoffatome aufweisen, mit einem Gemisch von

a) Alkoholen, ausgewählt aus der Gruppe bestehend aus
  - a1 gesättigten aliphatischen Alkoholen mit 4 bis 22 Kohlenstoffatomen,
  - a2 ungesättigten aliphatischen Alkoholen mit 8 bis 22 Kohlenstoffatomen,
  - a3 Alkylphenolen, deren Alkylgruppen jeweils 8 bis 12 Kohlenstoffatome aufweisen oder deren Oxalkylierungsprodukte mit 1 bis 3 Oxyethylen- und/oder Oxypropyleneinheiten und

b) Polyoxyalkylenmonoolen der allgemeinen, durchschnittlichen Formel

$$R^1O\text{-}(C_nH_{2n}O\text{-})_xH \qquad I$$

wobei

- $R^1$ der Kohlenwasserstoffrest eines Startalkohols $R^1OH$ ist,
- n 2, 3 oder 4 ist und in der durchschnittlichen Polyoxyalkylengruppe einen mittleren Wert von 2 bis 3 hat,
- x in der durchschnittlichen Polyoxyalkylengruppe einen mittleren Wert von 4 bis 50 hat,

wobei das molare Verhältnis von a : b und der Wert der Indices n und x so gewählt ist, daß das Verfahrensprodukt einen HLB-Wert von ≦ 8 aufweist, in solchen Mengen, daß bis zu 70 % der Estergruppen umgesetzt werden, in Gegenwart eines an sich bekannten Umesterungskatalysators bei Temperaturen von 70 bis 160 °C, gegebenenfalls in Gegenwart eines Lösungsmittels.

Es ist dabei dem Fachmann klar, daß der HLB-Wert des Polyacrylsäureesters auch von der Kettenlänge des Alkoholes a) abhängt.

Die hydrophoben Emulgatoren der Gruppe $a_2$) sind aus der EP-A-0 125 779 und der EP-A-0 176 884 bekannt. Es handelt sich hierbei um vorwiegend lineare Siloxane, welche seiten- und/oder endständig sowohl langkettige Alkylreste wie Polyetherreste aufweisen. Dabei ergibt sich der HLB-Wert dieser Verbindungen aus der Hydrophilie des Polyetherrestes oder der Polyetherreste, der Kettenlänge des Siloxans und der Kettenlänge des langkettigen Alkylrestes. Die modifizierten Polysiloxane können dabei der folgenden Formel entsprechen:

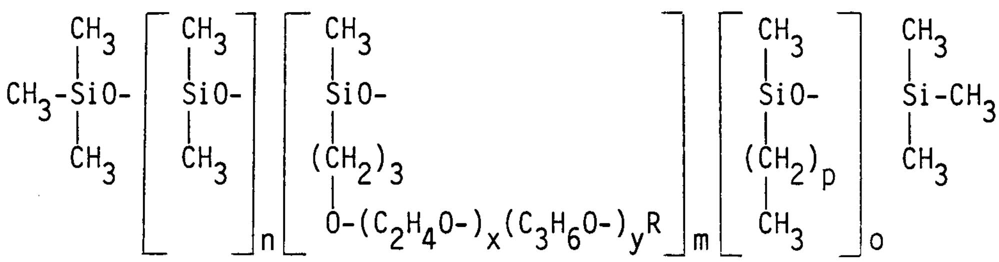

II

wobei

- R ein niederer Alkylrest oder ein Wasserstoffrest ist,
- n = 10 bis 200,
- m = 1 bis 25,
- o = 1 bis 100,
- p = 7 bis 17 und
- x und y ganze Zahlen von 1 bis 200 sind.

Die Kohlenstoffkette des langkettigen Alkylrestes kann durch einen Ethersauerstoff unterbrochen sein. Derartige Verbindungen sind in der EP-A-0 459 705 beschrieben.

Der an der Grenzfläche $O^1/W$ vorliegende hydrophile Emulgator soll nichtionogen sein und einen HLB-Wert > 8, vorzugsweise > 12, aufweisen. Geeignete nichtionogene Emulgatoren sind Polyoxyethylenether von Fettalkoholen, partiellen Fettsäureestern und Alkylphenolen oder Polyoxyethylenester von Fettsäuren.

Ein bevorzugter nichtionogener Emulgator ist der Polyoxyethylenester der Ricinolfettsäure.

Weitere bevorzugte hydrophile nichtionogene Emulgatoren sind

$b_1$) die partiellen Fettsäureester des Mono-, Oligo- oder Polyglycerins oder

$b_2$) die Polyacrylsäureester mit langkettigen Kohlenwasserstoffund Polyoxyalkylengruppen.

Die hydrophilen Emulgatoren des Typs $b_1$) sind Mono- oder Mono-/Di-ester des Glycerins oder partielle Ester, insbesondere des Di-, Tri-, Tetra- oder Pentaglycerins.

Ein partieller Monofettsäureester des Triglycerins entspricht zum Beispiel der Formel

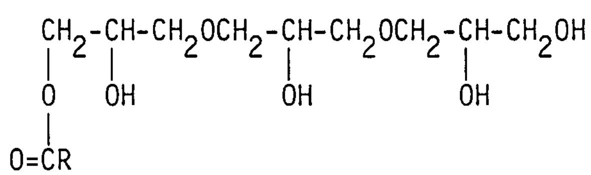

III

in der die Gruppe

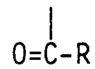

der Acylrest einer Fettsäure R-COOH ist.

Als Fettsäuren kommen insbesondere gesättigte Fettsäuren mit einer Kettenlänge von 12 bis 18 Kohlenstoffatomen in Betracht.

Zur Einstellung des gewünschten HLB-Wertes der hydrophilen Emulgatoren $b_1$) kann an eine oder mehrere der OH-Gruppen der partiellen Fettsäureester des Mono-, Oligo- oder Polyglycerins Ethylenoxid in entsprechender Menge angelagert werden.

Verwendet man Fettsäuremono- oder Fettsäuremono-/diester des Glycerins, können diese durch Zugabe von Alkalisalzen von Fettsäuren mit 10 bis 18 Kohlenstoffatomen in ihrem HLB-Wert erhöht werden. Derartige seifenhaltige partielle Glycerinfettsäureester sind selbstemulgierbar.

Der hydrophile Emulgator $b_2$) entspricht strukturell dem bereits beschriebenen hydrophoben Emulgator $a_1$), weist jedoch einen höheren Gehalt an Oxyethylengruppen auf, um den geforderten HLB-Wert von > 8 zu erbringen. Eine weitere Beeinflussung des HLB-Wertes ergibt sich durch die Variation der Kettenlänge des Siloxans und/oder der langkettigen Kohlenwasserstoffreste.

Der hydrophile und der hydrophobe Emulgator werden in Mengen von 0,5 bis 5 Gew.-%, bezogen auf Gesamtzubereitung, verwendet.

Die multiplen Emulsionen werden in an sich bekannter Weise hergestellt. Man stellt zunächst die innere Phase der multiplen Emulsion, d.h. die Emulsion vom Typ $O^1$/W her, indem man den hydrophilen Emulgator in der Wasserphase löst und das Öl in die wäßrige Phase einrührt. Es ist jedoch auch möglich, den Emulgator in der öligen Phase zu dispergieren und das Wasser einzurühren. Die erhaltene $O^1$/W-Emulsion kann gegebenenfalls durch Einwirkung von Scherkräften homogenisiert werden. In einem zweiten Schritt wird der hydrophobe Emulgator in der öligen Phase $O^2$ dispergiert. In diese Dispersion wird die $O^1$/W-Emulsion eingearbeitet.

Die Wirkstoffe, wie z.B. Vitamine, Pflanzen- und Organextrakte, Duftstoffe, pharmazeutisch wirksame Substanzen und dgl., werden vor der Emulgierung in der gewünschten Phase gelöst oder verteilt.

Die erfindungsgemäßen multiplen Emulsionen sind flüssig bis pastös und über mehrere Monate lagerstabil und deshalb handelsfähig. Ein besonderer Vorteil der erfindungsgemäßen Emulsionen besteht in ihrer Wärmestabilität.

In den folgenden Beispielen werden die Formulierung und Herstellung erfindungsgemäßer O/W/O-Emulsionen erläutert.

Daneben werden Vergleichsemulsionen gezeigt, welche bewährte, aber nicht erfindungsgemäße Emulgatoren enthalten und daher nicht ausreichend stabil sind.

Die Bildung der ternären Emulsionsform läßt sich indirekt durch Vergleiche mit Referenzproben beweisen, die infolge des Herstellungsverfahrens binären Charakter haben. Entsprechende Emulsionen werden ebenfalls zum Vergleich herangezogen.

1. Prinzipieller Aufbau der erfindungsgemäßen und nicht erfindungsgemäßen Emulsionen

1.1 Zusammensetzung der binären Vergleichsemulsionen

Binäre Vergleichsemulsion I:

Es handelt sich um binäre W/O-Emulsionen, deren äußere Ölphase ($O^2$) die gleiche Zusammensetzung und den gleichen Gewichtsanteil wie die multiplen haben, die jedoch als innere Phase anstelle der O/W-Emulsion ($O^1$/W) nur Wasser enthalten. Die ternäre und die binäre Vergleichsemulsion müssen in etwa die gleiche Viskosität haben, da für diese im wesentlichen der Gewichtsanteil und die Viskosität der äußeren Ölphase ($O^2$) verantwortlich sind.

Binäre Vergleichsemulsion II:

Es handelt sich um binäre W/O-Emulsionen, deren prozentuale Zusammensetzung mit den ternären identisch ist, in denen aber sämtliche lipidartigen Komponenten ($O^1$ und $O^2$) in der äußeren Phase enthalten sind. Dadurch wird das Verhältnis von äußerer zu disperser Phase zugunsten der äußeren verschoben. Das hat, im Vergleich zur ternären Emulsion, eine geringere Viskosität zur Folge.

1.2 Zusammensetzung der äußeren Ölphase ($O^2$)

Sie enthält die für kosmetische W/O-Emulsionen typischen Komponenten:

kosmetische Öle in Form von natürlichen oder synthetischen Fettsäureestern, Paraffinöle oder volatile Siliconöle;

Wachse, die zur Bildung emulsionsstabilisierender Oleogele befähigt sind, wie microkristalline Kohlenwasserstoffe (Microwachse), Bienenwachs oder gehärtetes Ricinusöl (Castorwachs);
lipophile, zur Herstellung von stabilen W/O-Emulsionen geeignete Emulgatoren; diese haben erfindungsgemäß polymeren Charakter.

Das siliciumorganische Copolymer, ein Polysiloxan-polycetyl-polyethylenglykol-copolymer (EM 90) hat, entsprechend Formel II, folgende Indices: R = H, n = 50, m = 4, o = 21, p = 15, x = 10, y = 0. Das derivatisierte Polymethylacrylat $a_1$) (PMAC-W/O) besteht aus 32 Acrylateinheiten, von denen 15 mit Methylgruppen, 15 mit Oleylgruppen und 2 mit Methoxypolyethylenglykolgruppen (MG 600) besetzt sind.

Zum Vergleich werden bewährte, jedoch nicht erfindungsgemäße W/O-Emulgatoren eingesetzt: Triglycerintrioleat (TGTO), Sorbitanmono-/dioleat (SMDO), Methylglucosedioleat (MGDO).

1.3 Aufbau der als disperse Phase fungierenden O/W-Emulsionen ($O^1$/W)

Es handelt sich um feinteilige O/W-Emulsionen, deren Ölphase ($O^1$) aus Pflanzenölen, wie Soja- oder Nachtkerzenöl bzw. handelsüblichem Sojalecithin, besteht.

Die Wasserphase enthält emulsionsstabilisierende Additive, wie die Elektrolyte Natriumchlorid oder Magnesiumsulfat und die wasserlöslichen Verdickungsmittel Natrium-Polyacrylat (Carbopol 1342) oder Xanthan Gum.

Die eingesetzten hydrophilen Emulgatoren sind: Ein durch Kaliumstearat selbstemulgierend eingestelltes 90 %iges Glycerinmonostearat (GMS SE), Methylglucosemono-/distearat (MGMS), Polyethylenglykol(60)-Ricinusöl (RZ 60) sowie ein hydrophiles derivatisiertes Polymethylacrylat (PMAC-O/W). Dieses basiert auf 32 Acrylateinheiten, davon sind 16 mit Methylgruppen, 9 mit Stearylgruppen und 7 mit Methoxypolyethylenglykolgruppen (MG 600) besetzt.

1.4 Herstellung der ternären O/W/O- und der W/O-Vergleichsemulsionen

Die Komponenten der äußeren Ölphase ($O^2$), d.h. Öle, Wachse und Emulgatoren werden auf ca. 80 °C erwärmt, um die Wachse in Lösung zu bringen. Anschließend wird, entsprechend der sogenannten Heiß-Kalt-Methode, die O/W-Emulsion ($O^1$/W) bzw. die wäßrige Lösung (Vergleichsemulsionen), deren Temperatur ca. 20 °C beträgt, unter Rühren eingearbeitet; dabei ist intensive Scherung erforderlich.

1.5 Prüfung der Emulsionsstabilität

Von kosmetischen und auch pharmazeutischen Emulsionspräparaten wird nicht nur Stabilität bei Raumtemperatur gefordert, sondern auch eine mehrmonatige Wärmebelastbarkeit bei 40 °C. Die geforderte Kältestabilität bis -5 °C ist leicht zu erfüllen, problematisch sind tiefere Temperaturen. Die Emulsionen von einigen der durchgeführten Versuchsreihen werden im Rahmen der Stabilitätsprüfungen zusätzlich drei Gefrier-Tau-Zyklen unterworfen. Ein Zyklus beinhaltet eine 24stündige Lagerung bei -15 °C und daran anschließend eine 24stündige Lagerung bei +20 °C.

2. Formulierungsbeispiele

2.1 O/W/O-Emulsionen mit Lecithin als innere Ölphase ($O^1$)

2.1.1 Die äußere Ölphase enthält neben den Emulgatoren natürliche Öle und Wachse bzw. damit verwandte Substanzen.

Als Lecithin wird ein handelsübliches Sojalecithin verwendet, das mittels Emulgator (GMS SE) und intensiver mechanischer Bearbeitung (Rotor-Stator-Homogenisator) bei ca. 60 °C in eine sehr feinteilige O/W-Emulsion ($O^1$/W) überführt wird.

Als W/O-Emulgatoren werden erfindungsgemäß das siliciumorganische Copolymer EM 90 und als Vergleich das Triglycerintrioleat verwendet. In den binären Referenzproben befindet sich das Lecithin in der externen Ölphase ($O^2$).

Ergebnis:

Die Emulsionen auf Basis des erfindungsgemäßen polymeren Emulgators (Beispiele 1 und 2), die das Lecithin emulsionsartig in der dispersen Phase enthalten, sind stabil. Der Beweis für die ternäre Struktur wird indirekt durch die Emulsionen (Beispiele 3 und 4) geliefert, die das Lecithin in der externen Ölphase enthalten. Diese sind zwar bei Raumtemperatur und in der Kälte stabil, zersetzen sich aber bei 40 °C sehr schnell. Die Vorteile des erfindungsgemäßen polymeren Emulgators beweisen die ternären Vergleichsemulsionen (Beispiele 5 und 6), die als Emulgator Triglycerintrioleat enthalten. Diese sind weder wärme- noch kältestabil.

2.1.2 Die äußere Ölphase ($O^2$) der folgenden Formulierungen enthält Paraffinöl und volatiles Siliconöl. Emulgatoren sind das erfindungsgemäße lipophile derivatisierte Polymethylacrylat (PMAC-W/O) bzw. zum Vergleich Methylglucosedioleat.

Ergebnis:

Der erfindungsgemäße polymere Emulgator ergibt stabile Emulsionen (Beispiele 7 und 8), während die mittels Methylglucosedioleat hergestellten (Beispiele 9 und 10) weder wärme- noch kältestabil

sind.

2.2 O/W/O-Emulsionen mit einem Pflanzenöl als innere Ölphase ($O^1$)

2.2.1 Die äußere Ölphase ($O^2$) besteht aus Emulgator, Wachsen, Paraffinöl bzw. natürlichen oder synthetischen Fettsäureestern. Emulgatoren sind das erfindungsgemäße siliciumorganische Copolymer und in den Vergleichsemulsionen das Triglycerintrioleat. Als innere Phase wird eine sehr feinteilige Sojaölemulsion verwendet. Sie enthält 1 % hydrophilen Emulgator in Form des PEG 60-Ricinusöls. Die Emulgierung erfolgt nach der Pastenmethode, einem dem Prinzip der Mayonnaiseherstellung entsprechenden Verfahren. Dieses hat den Vorteil, daß nur relativ niedrige Emulgatorzusätze benötigt werden. Es ist dadurch gekennzeichnet, daß das Öl in eine wäßrige Emulgatorlösung unter intensiver Scherung eingerührt wird.

Die sich bildende sehr feinteilige Emulsion, die aufgrund des hohen Gehaltes an disperser Ölphase (ca. 80 %) pastösen Charakter hat, wird anschließend mit Wasser verdünnt.

Ergebnis:

Die auf dem erfindungsgemäßen lipophilen Copolymer basierenden ternären Emulsionen (Beispiele 11 bis 14) sind stabil. Die ternäre Struktur wird durch die binären Vergleichsemulsionen (Beispiele 15 und 16) indirekt bewiesen. Diese stimmen in ihrer Zusammensetzung weitgehend mit den Beispielen 12 und 14 überein, im Unterschied zu diesen ist aber das Sojaöl der äußeren Ölphase ($O^2$) zugesetzt worden. Die Folge ist eine deutlich niedrigere Viskosität und eine ungenügende Stabilität. Die mit dem Triglycerintrioleat hergestellten ternären Vergleichsemulsionen (Beispiele 17 bis 20) sind nur bei 20 °C stabil, Wärme- und Kältebelastungen verursachen Zersetzung. Auch an diesen Beispielen werden die Vorteile der erfindungsgemäßen Formulierungen deutlich.

2.2.2 Die äußere Ölphase ($O^2$) der multiplen Emulsionen besteht aus Emulgator, Wachsen, Paraffinöl bzw. natürlichen oder synthetischen Fettsäureestern. Als W/O-Emulgatoren werden das erfindungsgemäße siliciumorganische Copolymer sowie vergleichsweise das Sorbitansesquioleat verwendet.

Die innere Phase $O^1$/W bildet eine feinteilige O/W-Nachtkerzenölemulsion. Als O/W-Emulgator dient das hydrophile derivatisierte Polymethylacrylat (PMAC-O/W). Die Emulgierung erfolgt bei 60 °C durch intensive mechanische Bearbeitung mittels Rotor-Stator-Homogenisator. Die Emulsionen werden vor der Weiterverarbeitung zur multiplen Form auf 20 °C abgekühlt. Im Unterschied zur Versuchsreihe 2.2.1 liegt die Dosierung des O/W-Emulgators höher, 2 % anstatt 1 %.

Ergebnis:

Die mit dem erfindungsgemäßen polymeren Emulgator hergestellten ternären Emulsionen (Beispiele 21 bis 23) sind stabil, während sich die mit Sorbitanmono-/dioleat gebildeten in der Wärme zersetzen (Beispiele 24 und 25).

2.1.1 O/W/O-Emulsionen, Lecithin

| | 1 | 2 | 3 | 4 | 5 | 6 |
|---|---|---|---|---|---|---|
| | % | % | % | % | % | % |
| TGTO | - | - | - | - | 3,0 | 3,0 |
| EM 90 | 2,0 | 2,0 | 2,0 | 2,0 | - | - |
| Bienenwachs | 1,2 | 1,2 | 1,2 | 1,2 | 1,8 | 1,8 |
| Castorwachs | 0,8 | 0,8 | 0,8 | 0,8 | 1,2 | 1,2 |
| Avocadoöl | 12,0 | - | 12,0 | - | 11,0 | - |
| $C_{8/10}$-Triglycerid | 12,0 | - | 12,0 | - | 11,0 | - |
| Jojobaöl | - | 12,0 | - | 12,0 | - | 11,0 |
| Decyloleat | - | 12,0 | - | 12,0 | - | 11,0 |
| Octylstearat | - | - | - | - | - | - |
| Sojalecithin | - | - | 2,5 | 2,5 | - | - |
| | (28,0) | (28,0) | (30,5) | (30,5) | (28,0) | (28,0) |
| Sojalecithin | 2,5 | 2,5 | - | - | 2,5 | 2,5 |
| GMS SE | 0,8 | 0,8 | - | - | 0,8 | 0,8 |
| Carbopol 1342 | 0,2 | 0,2 | 0,2 | 0,2 | - | - |
| Xanthan Gum | - | - | - | - | 0,4 | 0,4 |
| Natriumchlorid | 0,5 | 0,5 | 0,5 | 0,5 | - | - |
| Magnesiumsulfat | - | - | - | - | 0,4 | 0,4 |
| Wasser | 68,0 | 68,0 | 68,8 | 68,8 | 67,9 | 67,9 |
| | (72,0) | (72,0) | (69,5) | (69,5) | (72,0) | (72,0) |

Stabilität der Emulsionen

| | 1 Tg./20°C | 3 M/20°C | 3 M/40°C | 3x -15°C |
|---|---|---|---|---|
| Nr. 1 | p, s | p, s | p, s | p, s |
| Nr. 2 | fp, s | fp, s | fp, s | fp, s |
| Nr. 3 | p, s | p, s | z | p, s |
| Nr. 4 | fp, s | fp, s | z | p, s |
| Nr. 5 | p, s | p, s | z | z |
| Nr. 6 | p, s | p, s | z | z |

Erklärung:

Viskosität/Konsistenz

p = pastös
fp = fließfähig, hochviskos
f = flüssig, mittel-niedrigviskos

Stabilität

s = stabil
z = zersetzt, Öl- und/oder Wasserseparation

2.1.2 O/W/O-Emulsionen, Lecithin

| | 7 | 8 | 9 | 10 |
|---|---|---|---|---|
| | % | % | % | % |
| MGDO | - | - | 3,0 | 3,0 |
| PMAC-W/O | 2,0 | 2,0 | - | - |
| Microwachs (Fp > 70°) | 1,2 | 1,2 | 1,2 | 1,2 |
| Castorwachs | 0,8 | 0,8 | 0,8 | 0,8 |
| Paraffinöl (30 mPas) | 24,0 | - | 23,0 | - |
| Isohexadecan | - | 12,0 | - | 11,5 |
| Cyclomethylsiloxan ($D_5$) | - | 12,0 | - | 11,5 |
| | (28,0) | (28,0) | (28,0) | (28,0) |
| Sojalecithin | 2,5 | 2,5 | 2,5 | 2,5 |
| GMS SE | 0,8 | 0,8 | 0,8 | 0,8 |
| Natriumchlorid | 0,5 | 0,5 | 0,4 | 0,4 |
| Magnesiumsulfat | - | - | - | - |
| Wasser | 68,2 | 68,2 | 68,3 | 68,3 |
| | (72,0) | (72,0) | (72,0) | (72,0) |

Stabilität der Emulsionen

| | 1 Tg./20°C | 3 M/20°C | 3 M/40°C | 3x -15°C |
|---|---|---|---|---|
| Nr. 7 | fp, s | fp, s | fp, s | fp, s |
| Nr. 8 | fp, s | fp, s | fp, s | fp, s |
| Nr. 9 | p, s | p, s | z | z |
| Nr. 10 | p, s | p, s | z | z |

2.2.1 O/W/O-Emulsionen, Sojaöl

| | 11 | 12 | 13 | 14 | 15 | 16 | 17 | 18 | 19 | 20 |
|---|---|---|---|---|---|---|---|---|---|---|
| | % | % | % | % | % | % | % | % | % | % |
| EM 90 | 2,0 | 2,0 | 2,0 | 2,0 | 2,0 | 2,0 | - | - | - | - |
| TGTO | - | - | - | - | - | - | 4,0 | 4,0 | 4,0 | 4,0 |
| Bienenwachs | 1,2 | 1,2 | 1,2 | 1,2 | 1,2 | 1,2 | 1,8 | 1,8 | 1,8 | 1,8 |
| Castorwachs | 0,8 | 0,8 | 0,8 | 0,8 | 0,8 | 0,8 | 1,2 | 1,2 | 1,2 | 1,2 |
| Paraffinöl (30 mPas) | 24,0 | - | - | - | - | - | 21,0 | - | - | - |
| Octylstearat | - | 12,0 | - | - | 12,0 | - | - | 10,5 | - | - |
| $C_{8/10}$-Triglycerid | - | 12,0 | 12,0 | - | 12,0 | - | - | 10,5 | 10,5 | - |
| Avocadoöl | - | - | 12,0 | - | - | - | - | - | 10,5 | - |
| Jojobaöl | - | - | - | 12,0 | - | 12,0 | - | - | - | 10,5 |
| Decyloleat | - | - | - | 12,0 | - | 12,0 | - | - | - | 10,5 |
| Sojaöl | - | - | - | - | 10,0 | 10,0 | - | - | - | - |
| | (28,0) | (28,0) | (28,0) | (28,0) | (38,0) | (38,0) | (28,0) | (28,0) | (28,0) | (28,0) |
| RZ 60 | 1,0 | 1,0 | 1,0 | 1,0 | - | - | 1,0 | 1,0 | 1,0 | 1,0 |
| Sojaöl | 10,0 | 10,0 | 10,0 | 10,0 | - | - | 10,0 | 10,0 | 10,0 | 10,0 |
| Carbopol 1342 | 0,2 | 0,2 | 0,2 | 0,2 | 0,2 | 0,2 | - | - | - | - |
| Xanthan Gum | - | - | - | - | - | - | 0,5 | 0,5 | 0,5 | 0,5 |
| Natriumchlorid | 0,5 | 0,5 | 0,5 | 0,5 | 0,5 | 0,5 | - | - | - | - |
| Magnesiumsulfat | - | - | - | - | - | - | 0,4 | 0,4 | 0,4 | 0,4 |
| Wasser | 60,3 | 60,3 | 60,3 | 60,3 | 61,3 | 61,3 | 60,1 | 60,1 | 60,1 | 60,1 |
| | (72,0) | (72,0) | (72,0) | (72,0) | (62,0) | (62,0) | (72,0) | (72,0) | (72,0) | (72,0) |

2.2.2 O/W/O-Emulsionen, Nachtkerzenöl

| | 21 | 22 | 23 | 24 | 25 |
|---|---|---|---|---|---|
| | % | % | % | % | % |
| SMDO | - | - | - | 3,0 | 3,0 |
| EM 90 | 2,0 | 2,0 | 2,0 | - | - |
| Bienenwachs | 1,2 | 1,2 | 1,2 | 1,8 | 1,8 |
| Castorwachs | 0,8 | 0,8 | 0,8 | 1,2 | 1,2 |
| Paraffinöl (30 mPas) | 24,0 | - | - | 22,0 | - |
| $C_{8/10}$-Triglycerid | - | 12,0 | - | - | - |
| Octylstearat | - | 12,0 | - | - | - |
| Jojobaöl | - | - | 12,0 | - | 11,0 |
| Decyloleat | - | - | 12,0 | - | 11,0 |
| | (28,0) | (28,0) | (28,0) | (28,0) | (28,0) |
| PMAC-O/W | 2,0 | 2,0 | 2,0 | 2,0 | 2,0 |
| Nachtkerzenöl | 10,0 | 10,0 | 10,0 | 10,0 | 10,0 |
| Natriumchlorid | 0,5 | 0,5 | 0,5 | - | - |
| Magnesiumsulfat | - | - | - | 0,4 | 0,4 |
| Wasser | 59,5 | 59,5 | 59,5 | 59,6 | 59,6 |
| | (72,0) | (72,0) | (72,0) | (72,0) | (72,0) |

Stabilität der Emulsionen

| | 1 Tg./20°C | 3 M/20°C | 3 M/40°C |
|---|---|---|---|
| Nr. 21 | fp, s | fp, s | fp, s |
| Nr. 22 | p, s | p, s | p, s |
| Nr. 23 | p, s | p, s | p, s |
| Nr. 24 | p, s | p, s | z |
| Nr. 25 | p, s | z | z |

Stabilität der Emulsionen

| | 1 Tg./20°C | 3 M/20°C | 3 M/45°C | 5x -15°C |
|---|---|---|---|---|
| Nr. 11 | fp, s | fp, s | fp, s | fp, s |
| Nr. 12 | fp, s | fp, s | fp, s | fp, s |
| Nr. 13 | fp, s | fp, s | fp, s | fp, s |
| Nr. 14 | fp, s | fp, s | fp, s | fp, s |
| Nr. 15 | f, s | f, s | z | z |
| Nr. 16 | f, s | f, s | z | z |
| Nr. 17 | p, s | p, s | z | z |
| Nr. 18 | p, s | p, s | z | z |
| Nr. 19 | p, s | p, s | z | z |
| Nr. 20 | p, s | p, s | z | z |

**Patentansprüche**

1. Flüssige oder pastöse, lagerstabile, multiple Emulsion des Typs $O^1/W/O^2$, wobei $O^2$ die äußere Phase der multiplen Emulsion bildet, an der Grenzfläche $O^1/W$ ein hydrophiler Emulgator und an der Grenzfläche $W/O^2$ ein hydrophober Emulgator vorhanden ist, dadurch gekennzeichnet, daß
   (1) der hydrophobe Emulgator einen HLB-Wert ≦ 8 aufweist und
      $a_1$) ein Polyacrylsäureester mit langkettigen Kohlenwasserstoff- und Polyoxyalkylengruppen oder
      $a_2$) ein Polyoxyalkylenpolysiloxan mit an Si-Atomen gebundenen langkettigen Alkylresten ist, und
   (2) der hydrophile Emulgator ein nichtionogener Emulgator mit einem HLB-Wert > 8 ist.

2. Emulsion nach Anspruch 1, dadurch gekennzeichnet, daß der hydrophile Emulgator einen HLB-Wert > 12 aufweist.

3. Emulsion nach Anspruch 1 oder 2, dadurch gekennzeichnet, daß der hydrophile Emulgator
   $b_1$) ein partieller Fettsäureester des Mono-, Oligo- oder Polyglycerins oder
   $b_2$) ein Polyacrylsäureester mit langkettigen Kohlenwasserstoff- und Polyoxyalkylengruppen ist.

**4.** Emulsion nach Anspruch 3, dadurch gekennzeichnet, daß der Emulgator $b_1$) ein Polyoxyethylenester der Ricinolsäure ist.

**5.** Emulsion nach Anspruch 3 oder 4, dadurch gekennzeichnet, daß der Emulgator $b_1$) durch Zugabe eines Alkalisalzes einer Fettsäure selbstemulgierend ist.

## Claims

**1.** Liquid or paste-form multiple emulsion of the $O^1/W/O^2$ type with a long shelf life, where $O^2$ forms the outer phase of the multiple emulsion, a hydrophilic emulsifier is present at the $O^1/W$ interface and a hydrophobic emulsifier is present at the $W/O^2$ interface, characterized in that

(1) the hydrophobic emulsifier has an HLB value of ≦ 8 and is

$a_1$) a polyacrylate containing long-chain hydrocarbon and polyoxyalkylene groups or

$a_2$) a polyoxyalkylene-polysiloxane containing long-chain alkyl radicals bonded to Si atoms, and

(2) the hydrophilic emulsifier is a nonionogenic emulsifier having an HLB value of > 8.

**2.** Emulsion according to Claim 1, characterized in that the hydrophilic emulsifier has an HLB value of > 12.

**3.** Emulsion according to Claim 1 or 2 characterized in that the hydrophilic emulsifier is

$b_1$) a partial fatty acid ester of mono-, oligo- or polyglycerol or

$b_2$) a polyacrylate containing long-chain hydrocarbon and polyoxyalkylene groups.

**4.** Emulsion according to Claim 3, characterized in that the emulsifier $b_1$) is a polyoxyethylene ester of ricinoleic acid.

**5.** Emulsion according to Claim 3 or 4, characterized in that the emulsifier $b_1$) has been self-emulsified by addition of an alkali metal salt of a fatty acid.

## Revendications

**1.** Emulsion multiple, liquide ou pâteuse, stable pendant le stockage, du type $H^1/E/H^2$, où $H^2$ forme la phase externe de l'émulsion multiple, avec la présence d'un émulsifiant hydrophile à l'interface de $H^1/E$ et d'un émulsifiant hydrophobe à l'interface de $E/H^2$, caractérisée en ce que :

(1) l'émulsifiant hydrophobe a une valeur de HLB (équilibre hydrophile-lipophile) ≦ 8 et est formé de :

$a_1$) un polyacrylate avec des groupes d'hydrocarbures et des groupes de polyoxyalkylène à chaîne longue, ou

$a_2$) un polyoxyalkylènepolysiloxane avec des restes alkyles à chaîne longue liés aux atomes de silicium, et

(2) l'émulsifiant hydrophile est un émulsifiant non ionogène avec une valeur de HLB > 8.

**2.** Emulsion selon la revendication 1, caractérisée en ce que l'émulsifiant hydrophile a une valeur de HLB > 12.

**3.** Emulsion selon la revendication 1 ou 2, caractérisée en ce que l'émulsifiant hydrophile est :

$b_1$) un ester partiel d'acide gras et de mono-, oligoou polyglycérol, ou

$b_2$) un polyacrylate avec des groupes d'hydrocarbures et des groupes de polyoxyalkylène à chaîne longue.

**4.** Emulsion selon la revendication 3, caractérisée en ce que l'émulsifiant $b_1$) est un ester de polyoxyéthylène et de l'acide ricinoléique.

**5.** Emulsion selon la revendication 3 ou 4, caractérisée en ce que l'émulsifiant $b_1$) est auto-émulsifiant grâce à l'addition d'un sel alcalin d'un acide gras.